Europäisches Patentamt

European Patent Office

Office européen des brevets

(11) Veröffentlichungsnummer: **0 308 630 B1**

(12) **EUROPÄISCHE PATENTSCHRIFT**

(45) Veröffentlichungstag der Patentschrift: **28.10.92**

(21) Anmeldenummer: **88112511.6**

(22) Anmeldetag: **02.08.88**

(51) Int. Cl.5: **A61N 5/10**

(54) **Magnetkupplung.**

(30) Priorität: **23.09.87 DE 3731946**

(43) Veröffentlichungstag der Anmeldung:
**29.03.89 Patentblatt 89/13**

(45) Bekanntmachung des Hinweises auf die
Patenterteilung:
**28.10.92 Patentblatt 92/44**

(84) Benannte Vertragsstaaten:
**FR GB IT NL**

(56) Entgegenhaltungen:
**EP-A- 0 158 630**
**EP-A- 0 207 926**
**DE-A- 2 610 368**
**DE-B- 2 009 356**

(73) Patentinhaber: **ISOTOPEN-TECHNIK DR. SAU-
ERWEIN GMBH**
**Bergische Strasse 16**
**W-5657 Haan/Rheinl. 1(DE)**

(72) Erfinder: **Rohe, Karl-Heinz**
**Ohligserstrasse 85**
**W-5657 Haan(DE)**
Erfinder: **Sauerwein, Kurt, Dr.**
**Kattendahl 7**
**W-4006 Erkrath(DE)**
Erfinder: **Weinlich, Karl**
**Weberstrasse 18**
**W-5600 Wuppertal 2(DE)**
Erfinder: **Link, Rainer, dr.**
**Buchenhöhe 1**
**W-5014 Kerpen-Horrem(DE)**
Erfinder: **Kinzer, Norbert**
**Am Kohlenmeiler 155**
**W-5600 Wuppertal 22(DE)**

(74) Vertreter: **König, Reimar, Dr.-Ing. et al**
**Patentanwälte Dr.-Ing. Reimar König**
**Dipl.-Ing. Klaus Bergen Wilhelm-Tell-Strasse**
**14 Postfach 260162**
**W-4000 Düsseldorf 1(DE)**

# Beschreibung

Die Erfindung betrifft eine Magnetkupplung zwischen translatorisch beweglichen Körpern mit einem ein Kupplungselement aus einem permanentmagnetischen oder magnetisierbaren Material aufweisenden ersten Körper, einem wenigstens ein komplementäres Kupplungselement aus einem permanentmagnetischen oder magnetisierbaren Material aufweisenden zweiten Körper, einem ein mit dem zweiten Körper zusammenwirkendes Kupplungselement aus permanentmagnetischem oder magnetisierbarem Material aufweisenden dritten Körper und einem Abstandshalter zwischen dem komplementären Kupplungselement des zweiten Körpers und dem Kupplungselement des dritten Körpers.

Eine derartige Kupplung ist in der europäischen Offenlegungsschrift 0 207 926 im Zusammenhang mit einer lösbaren Kupplung zweier lichtleitender oder elektrisch leitender Bauteile miteinander beschrieben. Bei dieser Kupplung weist ein Bauteil einen Stift auf, der sich in eine Hülse einsetzen und in dieser geführt mit seiner Stirnfläche am anderen Bauteil zur Anlage bringen läßt. Dabei besteht der Stift zumindest teilweise aus magnetischem Material oder ist mit einem magnetischen Teil versehen, ist die Hülse als Magnet ausgebildet und von einem Magneten oder einer elektrischen Spule umgeben. Durch die magnetischen Teile wird die geforderte hohe Haltekraft der Kupplung erreicht, die jedoch unveränderlich ist, wenn Permanentmagnete verwendet werden. Besteht der die Hülse umgebende Magnet aus einer elektrischen Spule, läßt sich die Kupplung auch als Schalter verwenden.

Eine andere Kupplung ist in der europäischen Offenlegungsschrift 0 158 630 im Zusammenhang mit einer medizinisch therapeutischen Vorrichtung zum gefahrlosen Einbringen von radioaktiven Strahlenquellen in Bestrahlungsvorrichtungen beschrieben, die im menschlichen Körper eingebettet sind. Diese Vorrichtung besitzt zumindest eine Belade- und/oder Lagerstation für die Strahlenquellen und zumindest eine Fördervorrichtung für die Strahlenquellen; sie besteht aus einem oder mehreren identischen Modulen, die aus je einer abgeschirmten Belade- und/oder Lagerstation für die jeweiligen Strahlenquellen, je einer Fördervorrichtung und je einer in den menschlichen Körper einzubringenden, von der Fördervorrichtung abkoppelbaren Bestrahlungsvorrichtung besteht. Zu diesem Zweck ist zwischen der Strahlenquelle und der Fördervorrichtung eine in der Bestrahlungsposition die Verbindung freigebende bzw. lösbare Kupplung angeordnet. Diese Kupplung besteht aus Permanentmagneten bzw. magnetisierbaren Körpern, die mit der Fördervorrichtung verbunden sind und damit zusammenwirkenden Permanentmagneten bzw. magnetisierbaren Körpern an der Strahlenquelle. Diese bekannte Magnetkupplung soll sich im Bereich der Bestrahlungsvorrichtung durch Betätigen von federbelasteten Schiebern lösen lassen.

Da die Bestrahlungsvorrichtung implantiert wird, soll sie möglichst klein sein, keine irgendwie gearteten Betätigungsverbindungen nach außen aufweisen und bei der Betätigung kein Strahlenrisiko für das Krankenhauspersonal mit sich bringen.

Der Erfindung liegt daher die Aufgabe zugrunde, eine Magnetkupplung zwischen translatorisch beweglichen Körpern zu schaffen, die sich in der Endphase der translatorischen Bewegung ohne Zutun von außen kuppeln oder entkuppeln läßt.

Diese Aufgabe wird bei einer Magnetkupplung der eingangs erwähnten Art dadurch gelöst, daß der Abstandshalter willkürlich zwischen einer Arbeitsstellung mit geringstem Abstand und einem Größtwert der Kaltekraft zwischen dem komplementären Kupplungselement des zweiten Körpers und dem Kupplungselement des dritten Körpers und einer zweiten Arbeitsstellung mit einer vorgegebenen Mindesthaltekraft verstellbar ist, so daß in der ersten Arbeitsstellung die Kaltekraft zwischen dem komplementären Kupplungselement des zweiten Körpers und dem Kupplungselement des dritten Körpers und in der zweiten Arbeitsstellung die Haltekraft zwischen dem komplementären Kupplungselement des zweiten Körpers und dem Kupplungselement des ersten Körpers überwiegt.

Durch Verstellen des Abstandshalters läßt sich die Haltekraft zwischen dem komplementären Kupplungselement des zweiten Körpers und dem Kupplungselement des dritten Körpers zwischen einem Größtwert bei geringstem Abstand und einem vorgegebenen Mindestwert erreichen, der noch eine ausreichende Kupplungswirkung zwischen dem zweiten und dem dritten Körper gewährleistet. Wird nun der Abstandhalter so eingestellt, daß der zweite Körper am dritten Körper mit einer verhältnismäßig geringen Haltekraft angekuppelt ist, wird er vom Kupplungselement des ersten Körpers festgehalten, sobald der zweite Körper in den Bereich des ersten Körpers gelangt. Die Haltekraft des Kupplungselements des dritten Körpers reicht dann nicht mehr aus, um den zweiten Körper mit Hilfe des komplementären Kupplungselements aus dem Bereich des ersten Körpers fortzubewegen, so daß sich der dritte Körper vom zweiten Körper löst.

Soll nun der zweite Körper wieder aus dem Bereich des ersten Körpers fortbewegt werden, wird der Abstandhalter so verstellt, daß das Kupplungselement des dritten Körpers unmittelbar am komplementären Kupplungselement des zweiten Körpers anliegt. Jetzt überwiegt die Anziehungskraft zwischen diesen beiden Kupplungselementen gegenüber der Anziehungskraft zwischen dem komplementären Kupplungselement und dem Kupplungselement des ersten Körpers, so daß eine Bewegung des dritten Körpers den zweiten Körper aus dem Bereich des ersten Körpers fortbewegt. Die Kupplungselemente können entweder alle drei aus permanentmagnetischem Material bestehen. Ebenso ist es möglich nur zwei der drei Kupplungselemente aus permanentmagnetischem Material herzustellen, während das dritte Kupplungselement aus magnetisierbarem Material besteht. Schließlich ist es auch noch möglich, nur das komplementäre Kupplungselement aus magnetischem Material herzustellen und die beiden anderen Kupplungselemente aus magnetisierbarem Material zu fertigen.

In allen drei Fällen sind die Stärke der Magneten und der durch den Abstandhalter in beiden Arbeitsstellungen gegebene Abstand so vorgegeben, daß ein sicheres Kuppeln oder Lösen der Kupplung in den vorgegebenen Stellungen gewährleistet ist.

Vorzugsweise besteht der Abstandhalter aus einer den dritten Körper umfassenden verschiebbaren Hülse, deren beiden Arbeitsstellungen durch eine zwischen der Hülse und dem dritten Körper wirkende, mit einer vorgegebenen Kraft lösbaren Rastvorrichtung festgelegt werden.

Vorzugsweise besteht der erste Körper aus einer den zweiten Körper aufnehmenden Hülse, dessen eines Ende einen Anschlag für den Abstandhalter bildet. Auf diese Weise ist je nach der Stellung des Abstandhalters gewährleistet, daß sich das Kupplungselement des ersten Körpers und das komplementäre Kupplungselement des zweiten Körpers mit dem für das Kuppeln oder Entkuppeln notwendigen Abstand voneinander befinden.

Als Kupplungselemente dienen jeweils die einander zugewandten Enden der Körper.

Um in der dem ersten Körper abgewandten Endstellung des zweiten und des dritten Körpers ein Verstellen des Abstandhalters zu ermöglichen, ist im Bewegungspfad des dritten Körpers ein Gehäuse mit einem festen Anschlag und einem willkürlich beweglichen Anschlag für den Abstandhalter angeordnet. Wird der Körper mit dem Abstandhalter gegen den festen Anschlag geführt, so wird der Abstandhalter in die eine Arbeitsstellung verschoben, während das Verschieben in die andere Arbeitsstellung dadurch geschieht, daß der bewegliche Anschlag in den Bewegungspfad des Körpers mit dem Abstandhalter gebracht wird und der Körper mit dem Abstandhalter gegen diesen Anschlag bewegt wird. Auf diese Weise läßt sich die erfindungsgemäße Magnetkupplung stets so programmieren, daß sie den zweiten Körper bis zum ersten Körper bringt und dort beläßt oder aber vom ersten Körper löst und in die Ausgangsstellung zurückbringt.

Die erfindungsgemäße Magnetkupplung läßt sich besonders vorteilhaft verwenden, wenn der erste Körper Teil einer in den menschlichen Körper beispielsweise implantierten hohlen Bestrahlungsvorrichtung, der zweite Körper Teil einer in die Bestrahlungsvorrichtung verbringbaren Strahlenquelle ist und der dritte Körper sowie der Abstandhalter am Ende eines in einer Hülle geführten biegeweichen, aber schubsteifen Kabels befestigt sind.

Die Erfindung wird nachstehend anhand eines in der Zeichnung dargestellten Ausführungsbeispiels des näheren erläutert. In der Zeichnung zeigen:

Fig. 1 die erfindungsgemäße Magnetkupplung als Teil einer Vorrichtung zum gefahrlosen Einbringen radioaktiver Strahlenquellen in eine interstitiell eingebrachte Bestrahlungsvorrichtung in der Ausgangsposition,

Fig. 2 die Vorrichtung gemäß Fig. 1 in ihrer Endlage,

Fig. 3 die Vorrichtung gemäß Fig. 1 und 2 mit zurückgefahrener Fördervorrichtung und

Fig. 4 die Vorrichtung gemäß Fig. 1 bis 3 mit zum Zurückholen der Strahlenquelle vorgefahrener Fördervorrichtung.

Eine Vorrichtung zum Einbringen radioaktiver Strahlenquellen in eine interstitiell eingebrachte Bestrahlungsvorrichtung ist in der EP-OS 0 158 630 im einzelnen beschrieben, insbesondere das interstitielle Einbringen der Bestrahlungsvorrichtung in den menschlichen Körper und das Einbringen der radioaktiven Strahlenquellen in die Bestrahlungsvorrichtung.

In der Zeichnung sind daher nur die die erfindungsgemäße Magnetkupplung betreffenden Einzelheiten dargestellt.

Die in den menschlichen Körper eingebettete Bestrahlungsvorrichtung besteht aus einem als Hülse ausgebildeten Körper 1 mit einem am offenen Ende angeordneten Kupplungselement 2, das als ringförmiger Permanentmagnet ausgebildet ist.

Ein Körper 3 dient als Strahlenquelle, die in den Hohlraum der Hülse 1 verbracht werden soll, um im Inneren des menschlichen Körpers ihre Strahlungswirkung zu entfalten. Dieser Körper 3 weist an seinem einen Ende ein komplementäres Kupplungselement 4 auf, das ebenfalls aus einem Permanentmagneten besteht. Dieses komplementäre Kupplungselement 4 wirkt mit einem Körper 5 zusammen, dessen dem komplementären Kupplungselement 4 zugewandtes Ende ein weiteres Kupplungselement 6 in Form eines Permanentmagneten aufweist. Um den Körper 5 und das Kupplungselement 6 herum ist ein Abstandhalter 7 in Form einer verschiebbaren Hülse angeordnet, die sich zwischen zwei Arbeitsstellungen verschieben läßt. Zu diesem Zweck befindet sich zwischen dem Körper 5 und dem Kupplungselement 6 und der Hülse 7 eine Rastvorrichtung 8, z.B. in Form von in einer Bohrung des Körpers 5 angeordneten federbeaufschlagten Kugeln und in der Hülse 7 mit Abstand voneinander angeordneten Ringnuten.

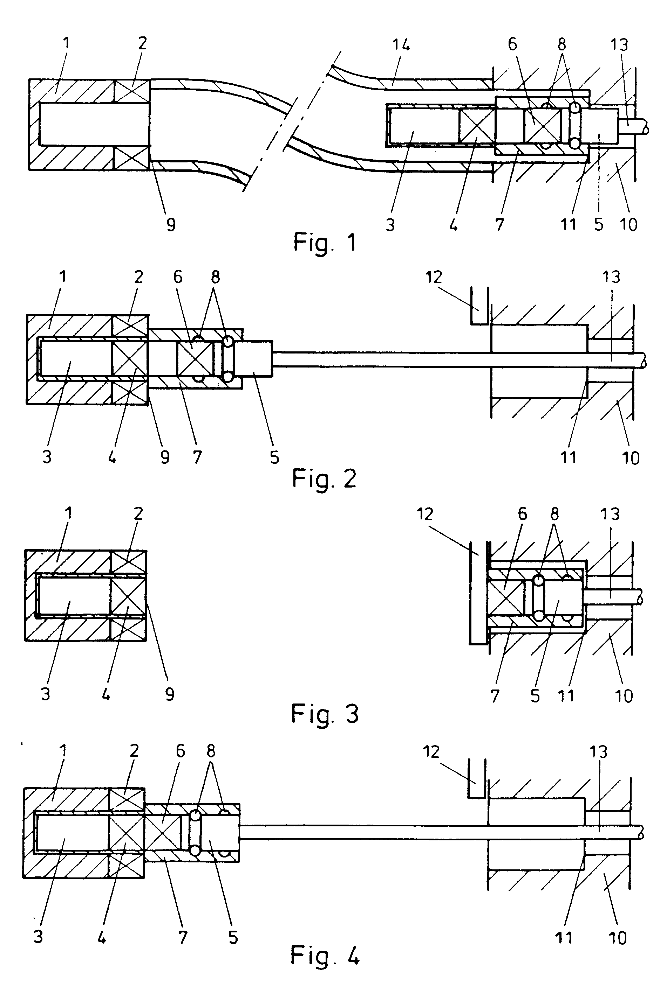

Eine Endfläche 9 des Kupplungselements 2 dient als Anschlag für die Hülse 7, wenn die miteinander gekuppelten Körper 3 und 5 mit Hilfe eines biegeweichen, jedoch schubsteifen in einer flexiblen Hülle 14 geführten Kabels 13 in die in Fig. 2 dargestellte Lage verschoben werden.

In den in Fig. 1 und 3 dargestellten Lagen befindet sich der Körper 5 mit dem Kupplungselement 6 und der Hülse 7 in einem Gehäuse 10, das mit einem festen Anschlag 11 und einem beweglichen Anschlag 12 für den Abstandhalter 7 versehen ist. In der in Fig. 1 dargestellten Lage ist der Körper 5 mit Hilfe des Kabels 13 soweit in das Gehäuse 10 hineingezogen, daß die Hülse 7 am festen Anschlag 11 in die in Fig. 1 dargestellte äußerste linke Arbeitsstellung verschoben wurde. Hierdurch ergibt sich zwischen dem Kupplungselement 4 und dem Kupplungselement 6 am Körper 5 ein Abstand bzw. Luftspalt, der ausreicht, um den Körper 3 mit dem Körper 5 zu kuppeln, so daß er sich in die in Fig. 2 dargestellte Lage verbringen läßt. In dieser Lage liegen sich die Kupplungselemente 2 und 4 so nahe gegenüber, daß ihre gegenseitige Anziehungskraft größer als die zwischen dem komplementären Kupplungselement 4 und dem Kupplungselement 6 ist. Wird nun der Körper 5 mit Hilfe des Kabels 13 zurückgezogen, trennen sich der Körper 3 und der Körper 5, hingegen verbleibt der Körper 3 in der Bestrahlungsvorrichtung 1, während der Körper 5 wieder in das Gehäuse 10 zurückgeführt wird. Wird nun, wie in Fig. 3 dargestellt, der bewegliche Anschlag 2 vor den Körper 5 mit dem Abstandhalter 7 geschoben, läßt er sich mit Hilfe des Kabels 13 in die in Fig. 3 dargestellte zweite Arbeitsstellung verschieben, bei der die Endflächen des Kupplungselements 6 und der Hülse 7 in einer Ebene liegen.

Soll nun die Strahlenquelle 3 wieder aus der Bestrahlungsvorrichtung 1 herausgezogen werden, wird der Körper 5 bis zum Kontakt zwischen dem komplementären Kupplungselement 4 und dem Kupplungselement 6 verschoben. Da das komplementäre Kupplungselement 4 und das Kupplungselement 6 nunmehr unmittelbar aneinander liegen, ist ihre gegenseitige Anziehungskraft größer als die zwischen dem komplementären Kupplungselement 4 und dem Kupplungselement 2, so daß sich die Strahlenquelle 3 durch Zurückbewegen des Kabels 13 aus der Bestrahlungsvorrichtung 1 holen läßt.

Auf diese Weise läßt sich die Bestrahlungsvorrichtung 1 völlig passiv mit geringem Volumen und ohne eine damit verbundene Betätigung gestalten, während sich das Umschalten der erfindungsgemäßen Magnetkupplung in erheblicher Entfernung von der Bestrahlungsvorrichtung 1, d.h. außerhalb des menschlichen Körpers durchführen läßt.

Nicht alle Kupplungselemente 2, 4, 6 brauchen aus Permanentmagneten zu bestehen, es kann genügen, daß nur das komplementäre Kupplungselement 4 oder zwei der Kupplungselemente 2, 4, 6 aus Permanentmagneten bestehen, während die übrigen Kupplungselemente aus einem magnetisierbaren Material gefertigt sind. Des weiteren ist es nicht erforderlich, daß sich die Kupplungselemente 4, 6 in der einen Arbeitsstellung berühren. Es reicht vielmehr aus, daß die Haltekraft zwischen dem komplementären Kupplungselement 4 und dem Kupplungselement 6 in der einen Arbeitsstellung geringer als die Haltekraft zwischen dem Kupplungselement 2 und dem komplementären Kupplungselement 4 und in der anderen Arbeitsstellung größer ist.

## Patentansprüche

1. Magnetkupplung zwischen translatorisch beweglichen Körpern mit einem ein Kupplungselement (2) aus einem permanentmagnetischen oder magnetisierbaren Material aufweisenden ersten Körper (1), einem wenigstens ein komplementäres Kupplungselement (4) aus einem permanentmagnetischen oder magnetisierbaren Material aufweisenden zweiten Körper (3), einem ein mit dem zweiten Körper (3) zusammenwirkendes Kupplungselement (6) aus permanentmagnetischen oder magnetisierbaren Material aufweisenden dritten Körper (5) und einem Abstandshalter (7) zwischen dem komplementären Kupplungselement (4) des zweiten Körpers (3) und dem Kupplungselement (6) des dritten Körpers (5), dadurch gekennzeichnet, daß der Abstandshalter (7) willkürlich zwischen einer Arbeitsstellung mit geringstem Abstand und einem Größtwert der Haltekraft zwischen dem komplementären Kupplungselement (4) des zweiten Körpers (3) und dem Kupplungselement (6) des dritten Körpers (5) und einer zweiten Arbeitsstellung mit einer vorgegebenen Mindesthaltekraft verstellbar ist, so daß in der ersten Arbeitsstellung die Kaltekraft zwischen dem komplementären Kupplungselement (4) des zweiten Körpers (3) und dem Kupplungselement (6) des dritten Körpers (5) und in der zweiten Arbeitsstellung die Haltekraft zwischen dem komplementären Kupplungselement (4) des zweiten Körpers (3) und dem Kupplungselement (2) des ersten Körpers (1) überwiegt.

2.  Magnetkupplung nach Anspruch 1, dadurch gekennzeichnet, daß alle drei Kupplungselemente (2, 4, 6) aus permanentmagnetischem Material bestehen.

3.  Magnetkupplung nach Anspruch 1, dadurch gekennzeichnet, daß wenigstens zwei der drei Kupplungselemente (2, 4, 6) aus permanentmagnetischem Material bestehen.

4.  Magnetkupplung nach Anspruch 1, dadurch gekennzeichnet, daß das komplementäre Kupplungselement (4) aus permanentmagnetischem Material und die beiden anderen Kupplungselemente (2, 6) aus magnetisierbarem Material bestehen.

5.  Magnetkupplung nach den Ansprüchen 1, 2, 3 oder 4, dadurch gekennzeichnet, daß der Abstandshalter (7) aus einer den dritten Körper (5) umfassenden, verschiebbaren Hülse (7) besteht.

6.  Magnetkupplung nach Anspruch 5, dadurch gekennzeichnet, daß zwischen der Hülse (7) und dem dritten Körper (5) eine die Hülse (7) in ihren beiden Arbeitsstellungen festhaltende, mit einer vorgegebenen Kraft lösbare Rastvorrichtung (8) angeordnet ist.

7.  Magnetkupplung nach einem oder mehreren der Ansprüche 1 bis 6, dadurch gekennzeichnet, daß der erste Körper (1) aus einer den zweiten Körper (3) aufnehmenden Hülse besteht, dessen eines Ende (9) einen Anschlag für den Abstandhalter (7) bildet.

8.  Magnetkupplung nach Anspruch 7, dadurch gekennzeichnet, daß das dem zweiten Körper (3) zugewandte Ende der Hülse (1) aus permanentmagnetischem oder magnetisierbarem Material besteht.

9.  Magnetkupplung nach Anspruch 8, dadurch gekennzeichnet, daß das dem dritten Körper (5) zugewandte Ende des zweiten Körpers (3) aus permanentmagnetischem oder magnetisierbarem Material besteht.

10. Magnetkupplung nach Anspruch 8 und 9, dadurch gekennzeichnet, daß das dem zweiten Körper (3) zugewandte Ende des dritten Körpers (5) aus permanentmagnetischem oder magnetisierbarem Material besteht.

11. Magnetkupplung nach den Ansprüchen 5 bis 10, dadurch gekennzeichnet, daß im Bewegungspfad des dritten Körpers (5) mit dem Abstandhalter (7) ein Gehäuse (10) mit einem festen Anschlag (11) und einem willkürlich beweglichen Anschlag (12) für den Abstandhalter (7) angeordnet ist.

12. Magnetkupplung nach den Ansprüchen 1 bis 11, dadurch gekennzeichnet, daß der erste Körper (1) Teil einer in den menschlichen Körper einzuführenden, hohlen Bestrahlungsvorrichtung und der zweite Körper (3) Teil einer in die Bestrahlungsvorrichtung verbringbaren Strahlenquelle ist und der dritte Körper (5) sowie der Abstandhalter (7) am Ende eines in einer Hülle (14) geführten biegeweichen, aber schubsteifen Kabels (13) befestigt sind.

## Claims

1.  A magnetic coupling between translationally movable bodies comprising a first body (1) having a coupling element (2) comprising a permanent magnetic or magnetisable material, a second body (3) having at least one complementary coupling element (4) comprising a permanent magnetic or magnetisable material, a third body (5) having a coupling element (6) comprising permanent magnetic or magnetisable material cooperating with the second body (3) and a spacer (7) between the complementary coupling element (4) of the second body (3) and the coupling element (6) of the third body (5), characterised in that the spacer (7) is adjustable as desired between an operating position with the smallest possible distance and a maximum value of the holding force between the complementary coupling element (4) of the second body (3) and the coupling element (6) of the third body (5) and a second operating position with a predetermined minimum holding force, so that in the first operating position the holding force between the complementary coupling element (4) of the second body (3) and the coupling element (6) of the third body (5) is the greater and in the second operating position the holding force between the complementary coupling element (4) of the second body (3) and the coupling element (2) of the first body (1) is the greater.

2.  A magnetic coupling according to claim 1, characterised in that all three coupling elements (2, 4, 6) consist of permanent magnetic material.

3.  A magnetic coupling according to claim 1, characterised in that at least two of the three coupling elements (2, 4, 6) consist of permanent magnetic material.

4. A magnetic coupling according to claim 1, characterised in that the complementary coupling element (4) consists of permanent magnetic material and the two other coupling elements (2, 6) consist of magnetisable material.

5. A magnetic coupling according to claim 1, 2, 3 or 4, characterised in that the spacer (7) consists of a displaceable sleeve (7) embracing the third body (5).

6. A magnetic coupling according to claim 5, characterised in that a snap-in locking device (8) holding the sleeve (7) in its two operating positions and releasable with a predetermined force is arranged between the sleeve (7) and the third body (5).

7. A magnetic coupling according to one or more of claims 1 to 6, characterised in that the first body (1) consists of a sleeve receiving the second body (3) and having one end forming a stop for the spacer (7).

8. A magnetic coupling according to claim 7, characterised in that the end of the sleeve (1) facing the second body (3) consists of permanent magnetic or magnetisable material.

9. A magnetic coupling according to claim 8, characterised in that the end of the second body (3) facing the third body (5) consists of permanent magnetic or magnetisable material.

10. A magnetic coupling according to claim 8 and claim 9, characterised in that the end of the third body (5) facing the second body (3) consists of permanent magnetic or magnetisable material.

11. A magnetic coupling according to any of claims 5 to 10, characterised in that a housing (10) having a fixed stop (11) and a stop (12) that can be moved as desired for the spacer (7) is arranged in the path of movement of the third body (5) with the spacer.

12. A magnetic coupling according to any of claims 1 to 11, characterised in that the first body (1) is part of a hollow irradiation device for insertion into the human body and the second body (3) is part of a radiation source that can be brought into the irradiation device and the third body (5) and the spacer (7) are fixed at the end of a flexible but compressively stiff cable (13) guided in a sleeve (14).

**Revendications**

1. Accouplement magnétique entre des organes déplaçables par translation, comportant un premier organe (1) équipé d'un élément d'accouplement (2) en un matériau à magnétisme permanent ou magnétisable, un deuxième organe (3) équipé d'au moins un élément d'accouplement complémentaire (4) en un matériau à magnétisme permanent ou magnétisable, un troisième organe (5) équipé d'un élément d'accouplement (6) en interaction avec le deuxième organe (3), en un matériau à magnétisme permanent ou magnétisable et un élément d'entretoisement (7) entre l'élément d'accouplement complémentaire (4) du deuxième organe (3) et l'élément d'accouplement (6) du troisième organe (5), caractérisé en ce que l'élément d'entretoisement (7) est déplaçable, sur commande, entre une position de travail qui présente la distance la plus faible et la valeur de force de retenue la plus élevée entre l'élément d'accouplement complémentaire (4) du deuxième organe (3) et l'élément d'accouplement (6) du troisième organe (5), et une deuxième position de travail qui présente une force de retenue la plus faible prédéterminée, de telle sorte que, dans la première position de travail, la force de retenue entre l'élément d'accouplement complémentaire (4) du deuxième (3) et l'élément d'accouplement (6) du troisième organe (5) l'emporte et que, dans la deuxième position de travail, la force de retenue entre l'élément d'accouplement complémentaire (4) du deuxième organe (3) et l'élément d'accouplement (2) du premier organe (1) l'emporte.

2. Accouplement magnétique selon la revendication 1 caractérisé en ce que les trois éléments d'accouplement (2,4,6) consistent en un matériau à magnétisme permanent.

3. Accouplement magnétique selon la revendication 1, caractérisé en ce que au moins deux des trois éléments d'accouplement (2,4,6) consistent en un matériau à magnétisme permanent.

4. Accouplement magnétique selon la revendication 1, caractérisé en ce que l'élément d'accouplement complémentaire (4) consiste en un matériau à magnétisme permanent et les deux autres éléments d'accouplement (2,6) consistent en un matériau magnétisable.

**5.** Accouplement magnétique selon la revendication 1,2,3 ou 4 caractérisé en ce que l'élément d'entretoisement (7) consiste en une douille déplaçable (7) qui enveloppe le troisième organe (5).

**6.** Accouplement magnétique selon la revendication 5 caractérisé en ce qu'un dispositif d'arrêt (8) qui est libérable moyennant une force prédéterminée, est agencé entre la douille (7) et le troisième organe (5) et maintient la douille (7) dans les deux positions de travail de celle-ci.

**7.** Accouplement magnétique selon l'une ou plusieurs des revendications 1 à 6 caractérisé en ce que le premier organe (1) consiste en une douille qui sert de logement au deuxième organe (3) et dont l'une des extrémités (9) constitue une butée pour l'élément d'entretoisement (7).

**8.** Accouplement magnétique selon la revendication 7 caractérisé en ce que l' extrémité de la douille (1), orientée vers le deuxième organe (3) consiste en un matériau à magnétisme permanent ou magnétisable.

**9.** Accouplement magnétique selon la revendication 8 caractérisé en ce que l'extrémité du deuxième organe (3), orientée vers le troisième organe (5) consiste en un matériau à magnétisme permanent ou magnétisable.

**10.** Accouplement magnétique selon les revendications 8 et 9 caractérisé en ce que l'extrémité du troisième organe (5) orientée vers le deuxième organe (3) consiste en un matériau à magnétisme permanent ou magnétisable.

**11.** Accouplement magnétique selon les revendications 5 à 10 caractérisé en ce qu'un boîtier (10) comportant une butée fixe (11) et une butée déplaçable sur commande (12) pour l'élément d'entretoisement (7) est agencé dans la trajectoire de déplacement du troisième organe (5) avec l'élément d'entretoisement (7).

**12.** Accouplement magnétique selon les revendications 1 à 11 caractérisé en ce que le premier organe (1) fait partie d'un dispositif d'irradiation creux à introduire dans le corps humain, le deuxième-organe (3) fait partie d'une source de rayonnement insérable dans le dispositif d'irradiation et le troisième organe (5) ainsi que l'élément d'entretoisement (7) sont fixés à l'extrémité d'un câble (13) flexible mais rigide à la poussée guidé dans une gaine (14).

Fig. 1

Fig. 2

Fig. 3

Fig. 4